# EUROPEAN PATENT APPLICATION

(11) **EP 3 699 594 A1**
(43) Date of publication of application: **26.08.2020**
(21) Application number: 19158840.9
(22) Date of filing: 22.02.2019
(51) Int. Cl.: G01N 33/574

(54) **USE OF BMMF1 REP PROTEIN AS A BIOMARKER FOR PROSTATE CANCER**

(71) Applicant: Deutsches Krebsforschungszentrum, Stiftung des öffentlichen Rechts, 69120 Heidelberg (DE)
(72) Inventor: Bund, Timo, 69221 Dossenheim (DE); de Villiers-zur Hausen, Ethel Michele, 69483 Waldmichelbach (DE); zur Hausen, Harald, 69483 Waldmichelbach (DE); Ernst, Claudia, 69436 Schönbrunn (DE); Tessmer, Claudia, 74869 Schwarzach (DE)
(74) Representative: Schüssler, Andrea

(57) **Abstract**

The present invention relates to the use of BMMF Rep-protein as biomarker for prostate cancer.

## Description

The invention relates to the use of a DNA-replication-associated (Rep) protein as a biomarker for prostate cancer.

### Background of the Invention

Prostate cancer is the second most common cause of cancer mortality in men in the United States. Over 200,000 new cases are identified each year and over 30,000 will die from this disease this year alone.

Most prostate cancer is initially androgen dependent, i.e. prostate cancer cells require androgen for continued proliferation. Androgen deprivation therapy (ADT) through either surgery or medical treatment rapidly leads to apoptosis of androgen-dependent cancer cells. ADT has been the mainstay of treatment for metastatic hormone sensitive prostate cancer (mHSPC) for more than 70 years.

In many cases, however, some cancer cells survive and become androgen independent or unresponsive, leading to recurrence of prostate cancer. Chemotherapy has been reserved for metastatic castration-resistant prostate cancer (mCRPC), a type of androgen-independent prostate cancer. Taxanes and DNA damaging agents are two major classes of chemotherapeutics used for treating prostate cancer.

Detection of prostate cancer early provides the best opportunity for a cure. Although prostate specific antigen (PSA) is considered as an effective tumor marker, it is not cancer specific. There is considerable overlap in PSA concentrations in men with prostate cancer and men with benign prostatic diseases. Furthermore, PSA levels cannot be used to differentiate men with indolent or organ confined prostate cancer (who would benefit from surgery) from those men with aggressive or non-organ confined prostate cancer (who would not benefit from surgery).

At present, serum PSA measurement, in combination with digital rectal examination (DRE), represents the leading tool used to detect and diagnose prostate cancer. Commercially available PSA assays are commonly performed in regional or local laboratories. These assays play apart in the current strategy for early detection of prostate cancer.

Because advanced disease is incurable, efforts have focused on identifying prostate cancer at an early stage, when it is confined to the prostate and therefore more amenable to cure. Unfortunately, prostate cancer can remain asymptomatic until tumor metastasis affects other organs or structures. Screening for prostate cancer is primarily done by the detection of PSA in the blood although the diagnostic value of PSA for prostate cancer is limited, due to its lack of specificity between benign and cancerous conditions. As mentioned above, PSA is not a disease-specific marker, as elevated levels of PSA are detectable in a large percentage of patients with benign prostatic hyperplasia (BPH) and prostatitis (25-86%),as well as in other nonmalignant disorders, which significantly limits the diagnostic specificity of this marker.

Thus, despite screening programs many patients are diagnosed late due to the lack of predictive biomarkers other than PSA. To enhance earlier detection, there is a need for biomarkers that will facilitate early detection and further insights into the pathogenesis of prostate cancer.

### Description of the Present Invention

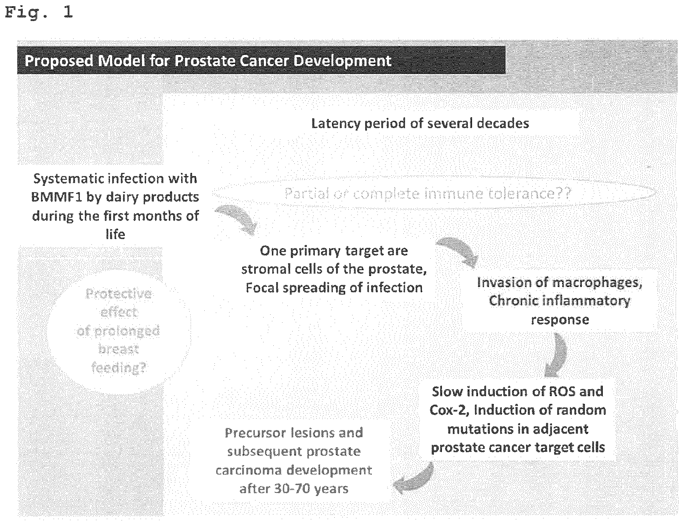

In the present application the inventors have created a model for prostate cancer development that is shown in Fig. 1.

The inventors found that the uptake of BMMF (Bovine Meat and Milk Factor) agents within the first months of life either by substitution of breast-feeding during weaning by cow milk products or by the uptake of dairy or beef products, in general, leads to the early infection of newborns with BMMF antigens. Based on the decline of maternal antibodies and the frequently observed weakness of the immune system often coupled with induction of immune tolerances of the newborn during this very early period of life, these agents might either directly escape immune response or a situation of immune tolerance against these agents might be induced. Within the next years or decades - depending on the immune system of the host - more and more BMMF antigens accumulate within the stroma of the prostate tissue. This accumulation may be triggered also by the uptake of specific molecules that may represent receptors for BMMFs. These molecules are also taken up by consumption of cow products and are metabolized into receptors on the surface of the host cells. When a certain level of antigen is reached by continuous uptake of BMMFs in combination with focal spreading of infection, the host immune response induces a state of chronic and local inflammation producing a stable increase of reactive oxygen species (ROS) and cyclooxygenase-2 (Cox-2) which dramatically increases the probability of deregulated cell proliferation with concomitant fixation of random mutations in surrounding cells induced by ROS. Especially, cells with intrinsically high replicative activity might represent targets enriching random DNA mutations enabling stochastic manifestation of mutations as a basic requirement for tumorgenesis and development of prostate cancer. Thus, BMMFs represent a specific and local trigger for induction of chronic inflammation within the tissue stroma leading to an increase of ROS which induces proliferation and mutation in surrounding replicative cells eventually leading to the formation of hyperplasia as precursors for cancer.

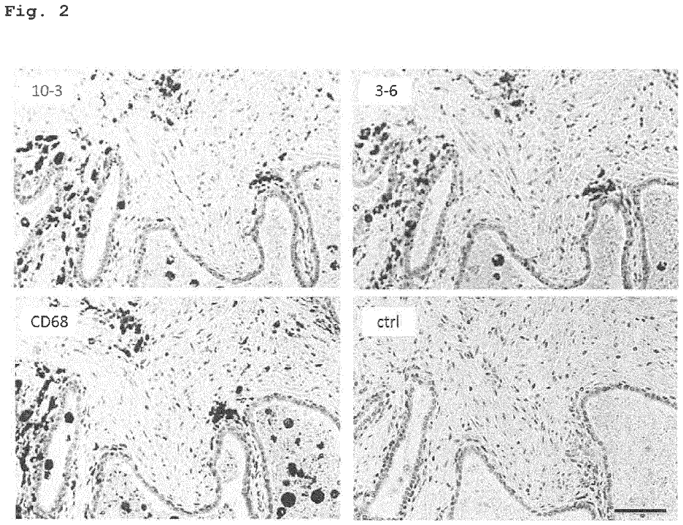

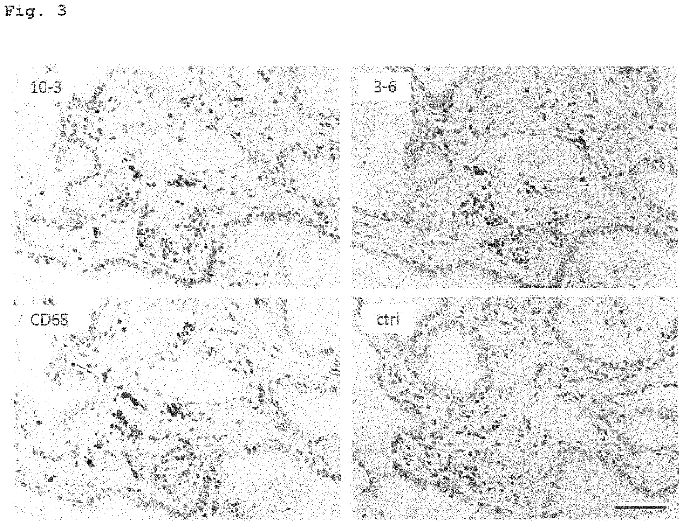

In detail, a selection of tissue samples from 12 prostate cancer patients with known tumor staging were subjected to IHC staining with mouse monoclonal anti-Rep antibodies. All tissues were tested positive for BMMF1 Rep targets. Exemplarily, the staining with anti-Rep antibodies (e.g. mAb 10-3, mAb 3-6) shows specific detection of protein targets in stromal tumor tissue regions within prostate cancer patient samples 17AD97 and 16RAV2 (Fig. 2 and 3). In general, the anti-Rep detection resulted in intense staining of smaller sized aggregates mainly within the cytoplasmic regions of cells within the stroma. Additionally, a colocalization of the anti-Rep stained signals with CD68-positive macrophages was observed. The regions with highest Rep-specific antibody detection correlate with regions with highest detection levels for CD68 positive cells pointing towards a localization of the Rep-specific antigens in inflammatory tissue areas, i.e. regions with especially high levels of inflammatory monocytes, circulating macrophages, or resident tissue macrophages. No signal detection was observed in control stainings with an antibody isotype control. On the other hand, significant anti-Rep staining patterns were also observed in epithelial cells surrounding the walls of prostate ducts and acini with aggregate-like cytoplasmic localization, which might represent tissue areas enabling BMMF replication/persistence.

So far a spectrum of 18 different, but partially related, DNA molecules were isolated from different test material (bovine sera, milk, brain tissue of one multiple sclerosis patient autopsies) (Funk, Gunst et al. 2014, Gunst, zur Hausen et al. 2014, Lamberto, Gunst et al. 2014, Whitley, Gunst et al. 2014; WO 2015/062726 A2; WO 2016/005054 A2). The 18 isolates were divided into four different groups BMMF1 through BMMF4, according to their molecular characteristics (zur Hausen et al., 2017). Three of these groups revealed a remarkable degree of similarity to *Acinetobacter baumannii* and Psychrobacter plasmids. The fourth group comprised 3 isolates being representatives of *Gemycirularviridae.* Putative Rep genes were identified as part of the BMMF's DNA sequences obtained by *in silico* comparisons to available sequences. Amplification using abutting primers in the rep gene led to the isolation of full and partial circular DNA genomes from bovine sera (Funk et al., 2014). This was extended to samples from commercially available milk products for the presence of specific circular single-stranded DNA genomes. Full-length circular single-stranded DNA molecules of 14 different isolates of (∼1100 to 3000 nucleotides) were cloned and sequenced (Whitley et al., 2014; Gunst et al., 2014; Funk et al., 2014; Lamberto et al., 2014). Four additional isolates were obtained from human brain and serum (all from patients with multiple sclerosis) (Whitley et al., 2014; Gunst et al., 2014; Lamberto et al., 2014).

Among these isolates two DNA molecules closely related to transmissible spongiform encephalopathy (TSE)-associated isolate Sphinx 1.76 (1,758 bp; accession no. HQ444404, (Manuelidis L. 2011)) were isolated from brain tissue from an MS patient. These isolates were MSBI1.176 (MSBI, multiple sclerosis brain isolate) (1,766 bp) and MSBI2.176 (1,766 bp) which are designated as "MSBI1 genome" and "MSBI2 genome", respectively. MSBI1.176 shares 98% nucleotide similarity to the sequence of Sphinx 1.76. The large open reading frames (ORFs) of the isolates encode a putative DNA replication protein sharing high similarity between them. Another common feature is the presence of iteron-like tandem repeats. The alignment of this repeat region indicates a variation in the core of single nucleotides. This iteron-like repeats may constitute the binding sites for Rep proteins. The sequences of the isolates have been deposited in the EMBL Databank under accession numbers LK931491 (MSBI1.176) and LK931492 (MSBI2.176) (Whitley C. et al. 2014) and have been aligned and described in WO 2016/005054 A2.

Further isolates were obtained from cow milk. These Cow milk isolates (CMI) were CMI1.252, CMI2.214 and CMI3.168 which are designated as "CMI1 genome", "CMI2 genome" and "CMI3 genome", respectively. The sequences of the isolates have been deposited in the EMBL Databank under accession numbers LK931487 (CMI1.252), LK931488 (CMI2.214) and LK931489 (CMI3.168) and have been aligned and described in WO 2016/005054 A2.

The present inventors have found that both CMI genomes and MSBI genomes show a significant production of transcribed RNA and the encoded Rep protein is expressed mostly in peripheral tissue around the cancer tissue The present inventors have found that the encoded Rep proteins (MSBI1 Rep, MSBI2 Rep, CMI1 Rep, CMI2 Rep, CMI3 Rep) represent a biomarker for prostate cancer. As DNA-replication-associated protein (RepB) the Rep protein has DNA binding activity and can be essential for initiation of replication of episomal or viral DNA molecules. Rep proteins show a marked potential of self-oligomerization and aggregation, which was described within prokaryotic systems *in vivo* and *in vitro* (Giraldo, Moreno-Diaz de la Espina et al. 2011, Torreira, Moreno-Del Alamo et al. 2015).

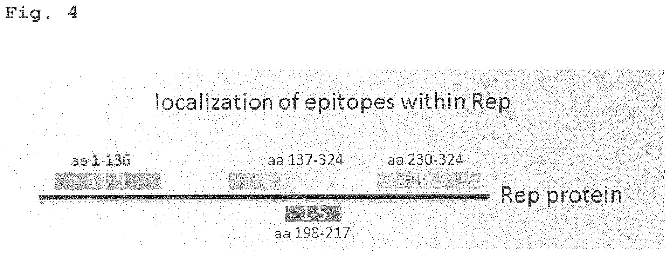

The inventors have raised monoclonal antibodies against Rep protein. In particular embodiments the anti-Rep antibodies bind to epitopes of Rep protein that are exemplified in Fig. 4. Particular preferred antibodies bind to epitopes within an amino acid sequence selected from the group consisting of amino acids from 1 to 136, from 137 to 229 and from 230 to 324 of SEQ ID NO:1. For example, the antibody binds to an epitope comprised by SEQ ID NO:2 or SEQ ID NO:3.

### Brief description of the drawings:

- Figure 1: shows the proposed model for prostate cancer development
- Figure 2: IHC detection of BMMF1 Rep on prostate cancer patient tissue 17AD97 (scale bar = 100 µm) in consecutive tissue sections.
- Figure 3: IHC detection of BMMF1 Rep on prostate cancer patient tissue 16RAV2 (scale bar = 100 µm) in consecutive tissue sections.
- Figure 4: shows characteristics of the raised antibodies and the localization of epitopes within Rep

The invention provides the teaching that Rep proteins may represent biomarkers for an enhanced risk to develop prostate cancer and are useful as a marker for determining the overall survival prognosis of prostate cancer patients.

The term "prostate cancer" means a malignant tumor that evolved as a consequence of uncontrolled cell growth in the prostate. These malignancies may develop as a consequence of pre-existing benign hyperplasias where genetic alterations promote the transition from normal to cancerous growth. The term "prostate cancer" means pre-stages, early stages or late stages of the disease and metastases derived therefrom.

In an alternative embodiment the present invention may also encompass the systematic testing of healthy prostate tissue (tissue from individuals without cancer diagnosis or a specific hint for the disease) to assess the disease risk in the future. This means that the present invention is also suitable to determine the predisposition for developing prostate cancer.

"Rep protein" as used herein refers to a DNA-replication-associated protein (RepB). The Rep protein comprises DNA binding activity and could be essential for initiation of replication of episomal/viral DNA molecules. In general Rep protein refers to a Rep protein from the group of the Small Sphinx Genome (Whitley et al., 2014). In particular, the Rep protein is a MSBI1 genome-encoded Rep protein (MSBI1 Rep), a MSBI2 genome-encoded Rep protein (MSBI2 Rep), a CMI1 genome-encoded Rep protein (CMI1 Rep), a CMI2 genome-encoded Rep protein (CMI2 Rep) or CMI3 genome-encoded Rep protein (CMI3 Rep). Preferably, the MSBI1 Rep protein is encoded by MSBI1.176 deposited in the EMBL databank under the acc. no. LK931491 and has the amino acid sequence as depicted in SEQ ID NO:1 or the Rep protein is MSBI2 encoded by MSBI2.176 deposited in the EMBL databank under the acc. no. LK931492 and has the amino acid sequence as depicted in SEQ ID NO:8 (Whitley, Gunst et al. 2014). In another preferred embodiment the CMI1 Rep protein is encoded by CMI1.252 deposited in the EMBL databank under the acc. no. LK931487 and has the amino acid sequence as depicted in SEQ ID NO:10. In another preferred embodiment the CMI2 Rep protein is encoded by CMI2.214 deposited in the EMBL databank under the acc. no. LK931488 and has the amino acid sequence as depicted in SEQ ID NO:11. In another preferred embodiment the CMI3 Rep protein is encoded by CMI3.168 deposited in the EMBL databank under the acc. no. LK931489 and has the amino acid sequence as depicted in SEQ ID NO:12. In a particular preferred embodiment the Rep protein comprises a N-terminal region conserved among BMMF1 genomes consisting essentially of amino acids from 1 to 229 of SEQ ID NO:1 and a C-terminal variable region specific for MSBI1.176 consisting essentially from amino acids 230 to 324 of SEQ ID NO:1. The N-terminal conserved region comprises a putative, first DNA binding domain consisting essentially of amino acids from 1 to 136 of SEQ ID NO: 1 and a second putative DNA binding domain consisting essentially of amino acids from 137 to 229 of SEQ ID NO:1. The C-terminal domain shows little sequence homology with any known protein and consists of amino acids 230 to 324.

"Rep protein" also encompasses fragments and variants of the protein with SEQ ID NO:1 or SEQ ID NO:8 which are capable of binding an anti-Rep antibody specific for Rep protein having the amino acid sequence of SEQ ID NO:1 or SEQ ID NO:8. Preferably, such a fragment is an immunogenic fragment of the protein having the amino acid sequence of SEQ ID NO:1 or SEQ ID NO:8 which encompasses at least one epitope for an anti-Rep protein antibody against the Rep protein of SEQ ID NO:1 or SEQ ID NO:8 and, preferably, comprises at least 7, 8, 9, 10, 15, 20, 25 or 50 contiguous amino acids. In particular embodiments the fragment comprises or consists essentially of a domain of the Rep protein, for example, the N-terminal conserved region, the C-terminal variable region, the first or second DNA binding domain. A variant of the protein with SEQ ID NO:1 or SEQ ID NO:8 comprises one or more amino acid deletions, substitutions or additions compared to SEQ ID NO:1 and has a homology of at least 90%, 91%, 92%, 93%, 94%, 95%, 96%, 97%, 98% or 99% to the amino acid sequence of SEQ ID NO:1 or SEQ ID NO:8, wherein the variant is capable of binding an anti-Rep antibody specific for a Rep protein having the amino acid sequence of SEQ ID NO:1 or SEQ ID NO:8. Included within the definition of variant are, for example, polypeptides containing one or more analogues of an amino acid (including, for example, unnatural amino acids, peptide nucleic acid (PNA), etc.), polypeptides with substituted linkages, as well as other modifications known in the art, both naturally occurring and non-naturally occurring. The term Rep protein includes fusion proteins with a heterologous amino acid sequence, with a leader sequence or with a Tag-sequence and the like. In certain embodiments of the invention protein tags are genetically grafted onto the Rep protein described above, for example the Rep protein selected from the group consisting of MSBI1, MSBI2, CMI1, CMI2 or CMI3. In particular at least one protein tag is attached to a polypeptide consisting of an amino acid sequence as depicted in any one of SEQ ID NOs:1-3,8-12,14. Such protein tags may be removable by chemical agents or by enzymatic means. Examples of protein tags are affinity or chromatography tags for purification. For example the Rep protein may be fused to a Tag-sequence, for example, selected from the group consisting of His₆-Tag (SEQ ID NO:4), T7-Tag (SEQ ID NO:5), FLAG-Tag (SEQ ID NO:6)and Strep-II-Tag (SEQ ID NO:7). a His-Tag (SEQ ID No:4), a T7-Tag (SEQ ID NO:5), FLAG-Tag (SEQ ID NO:6) or Strepll-Tag (SEQ ID NO:7). Further, fluorescence tags such as green fluorescence protein (GFP) or its variants may be attached to a Rep-protein according to the invention.

In a particular preferred embodiment the MSBI1 genome-encoded Rep protein (MSBI1 Rep) is codon-optimized for the production in human cell lines (e.g. HEK-293, HEK293TT, HEK293T, HEK293FT, HaCaT, HeLa, SiHa, CaSki, HDMEC, L1236, L428, BJAB, MCF7, Colo678, any primary cell lines) as well as bovine (e.g. MAC-T) or murine cell lines (e.g. GT1-7). This is described in detail in PCT/EP2017/075774.

The Rep protein of the invention, including the Rep fragments and Rep variants as defined above, can be prepared by classical chemical synthesis. The synthesis can be carried out in homogeneous solution or in solid phase. The polypeptides according to this invention can also be prepared by means of recombinant DNA techniques.

"Subject" as used herein refers to a mammalian individual or patient, including murines, cattle, for example bovines, simians and humans. Preferably, the subject is a human patient.

"Anti-Rep antibody" as used herein refers to an antibody binding at a detectable level to Rep protein which affinity is more strongly to the Rep protein of the invention than to a non-Rep protein. Preferably, the antigen affinity for Rep protein is at least 2 fold larger than background binding. In particular the anti-Rep antibody is specific for the MSBI1 Rep having the amino acid sequence of SEQ ID NO:1 or MSBI2 Rep. In particular embodiments the antibody is cross-specific for MSBI1 Rep, MSBI2 Rep, CMI1 Rep, CMI2 Rep and/or CMI3 Rep. In certain embodiments the anti-Rep antibody is cross-specific for at least two, preferably all, off MSBI1 Rep, MSBI2 Rep, CMI1 Rep, CMI2 Rep and/or CMI3 Rep.

The inventors also tested the antibody level of prostate cancer patients by contacting the Rep protein with a specimen suspected of containing anti-Rep protein antibodies under conditions that permit the Rep protein to bind to any such antibody present in the specimen. Such conditions will typically be physiologic temperature, pH and ionic strength using an excess of Rep protein. The incubation of the Rep protein with the specimen is followed by detection of immune complexes comprised of the antigen. In certain embodiments either the Rep protein is coupled to a signal generating compound, e.g. detectable label, or an additional binding agent, e.g. secondary antihuman antibody, coupled to a signal generating compound is used for detecting the immune complex.

Anti-Rep antibodies can be detected and quantified in assays based on Rep protein as protein antigen, which serves as target for the mammalian, e.g. human, antibodies suspected in the specimen. Preferably, the Rep protein is purified and the specimen can be, for example, serum or plasma. The methods include immobilization of Rep protein on a matrix followed by incubation of the immobilized Rep protein with the specimen. Finally, the Rep-bound antibodies of the formed immunological complex between Rep protein and antibodies of the specimen are quantified by a detection binding agent coupled to a signal generating compound, e.g. secondary HRP-(horseradish-peroxidase)-coupled detection antibody allowing for HRP-substrate based quantification. This signal generating compound or label is in itself detectable or may be reacted with an additional compound to generate a detectable product.

Design of the immunoassay is subject to a great deal of variation, and many formats are known in the art. Protocols may, for example, use solid supports, or immunoprecipitation. Most assays involve the use of binding agents coupled to signal generating compounds, for example labelled antibody or labelled Rep protein; the labels may be, for example, enzymatic, fluorescent, chemiluminescent, radioactive, or dye molecules. Assays which amplify the signals from the immune complex are also known; examples of which are assays which utilize biotin and avidin or streptavidin, and enzyme-labeled and mediated immunoassays, such as ELISA assays.

The immunoassay may be in a heterogeneous or in a homogeneous format, and of a standard or competitive type. Both standard and competitive formats are known in the art.

In an immunoprecipitation or agglutination assay format the reaction between the Rep protein and the anti-Rep antibody forms a network that precipitates from the solution or suspension and forms a visible layer or film of precipitate. If no anti-Rep antibody is present in the specimen, no visible precipitate is formed.

In further embodiments the inventors used methods wherein an increased amount of Rep protein in a sample correlates with a diagnosis or predisposition of prostate cancer. In such embodiments the Rep protein in the sample is detected by anti-Rep antibodies.

"Sample" as used herein refers to a biological sample encompassing cancerous prostate tissue, peripheral tissue surrounding the cancerous tissue and (benign) hyperplasias. The samples encompass tissue samples such as tissue cultures or biopsy specimen.

Such methods comprise the steps of detecting Rep protein in a sample from a subject by anti-Rep antibodies. In such methods Rep protein is detected in tissue samples by immunohistochemical methods or immunofluoresence microscopy.

In certain embodiments anti-Rep antibodies are used for the detection or capturing of the Rep protein in the sample.

The term "antibody", preferably, relates to antibodies which consist essentially of pooled polyclonal antibodies with different epitopic specificities, as well as distinct monoclonal antibody preparations. As used herein, the term "antibody"(Ab) or "monoclonal antibody" (Mab) is meant to include intact immunoglobulin molecules as well as antibody fragments (such as, for example, Fab and F(ab')2 fragments) which are capable of specifically binding to Rep protein. Fab and F(ab')2 fragments lack the Fc fragment of intact antibody, clear more rapidly from the circulation, and may have less non-specific tissue binding than an intact antibody. Thus, these fragments are preferred, as well as the products of a FAB or other immunoglobulin expression library. Moreover, antibodies useful for the purposes of the present invention include chimeric, single chain, multifunctional (e.g. bispecific) and humanized antibodies or human antibodies.

In certain embodiments the antibody or antigen binding fragment thereof is coupled to a signal generating compound, e.g., carries a detectable label. The antibody or antigen binding fragment thereof can be directly or indirectly detectably labeled, for example, with a radioisotope, a fluorescent compound, a bioluminescent compound, a chemiluminescent compound, a metal chelator or an enzyme. Those of ordinary skill in the art will know of other suitable labels for binding to the antibody, or will be able to ascertain such, using routine experimentation.

Anti-Rep antibodies are, preferably, raised (generated) against a Rep protein having the amino acid sequence of SEQ ID NO:1 or SEQ ID NO:8 or a fragment thereof by methods well known to those skilled in the art.

In certain embodiments anti-Rep antibodies are used in the methods of the invention which are capable of binding to several or all kinds of Rep proteins from the group of the Small Sphinx Genome (anti-Small-Sphinx-like Rep antibody or anti-SSLRep antibody). Such anti-SSLRep antibody binds to an epitope within the conserved N-terminal region of the Rep protein from amino acids 1 to 229 of SEQ ID NO:1. In particular embodiments anti-Rep antibodies of the anti-SSLRep type are used which bind to an epitope within SEQ ID NO:2 (amino acids 32-49 of SEQ ID NO:1) or SEQ ID NO:3 (amino acids 197-216 of SEQ ID NO:1). The peptide fragments of SEQ ID NO:2 and SEQ ID NO:3 are highly conserved among the Rep proteins from the Small Sphinx Genome group and appear to be exposed due to their hydrophilic character. Anti-Rep antibodies of the anti-SSLRep type may be produced by immunization, for example of mice or guinea pig, by peptides consisting essentially of the amino acid sequences as depicted in SEQ ID NOs:2 or 3; or by other immunogenic fragments, preferably comprising at least 8-15 amino acids, derived from the conserved N-terminal Rep protein region from amino acids 1 to 229 of SEQ ID NO:1.

In further embodiments anti-Rep antibodies specific for MSBI1 Rep protein are used. Such antibodies may be produced, for example, by immunization of a mammal such as mice or guinea pig with a full-length Rep protein having the amino acid sequence of SEQ ID NO:1.

Preferably, the methods of the invention use anti-Rep antibodies which are capable of detecting Rep protein up to ranges from picogramm to femtogramm.

Examples of such groups of anti-Rep antibodies are shown in Table 1:

| **Antibody Group** | **Rep-Protein Localisation** | **Specificity** | **Antibody** | **DSMZ deposit** |
|---|---|---|---|---|
| **Group A** | cytoplasm + nuclear membrane (+nucleus) | MSBI1 + small-sphinx-like All BMMF1 Reps | AB01 523-1-1 (Ab 1-5) | DSM ACC3327 |
| **Group B** | speckles in cytoplasm | MSBI1 + small-sphinx-like | AB02 304-4-1 (Ab 5-2) | DSM ACC3328 |
| **Group C** | cytoplasm + nuclear membrane (+ nucleus) | MSBI1 specific | MSBI1 381-6-2 (Ab 3-6) | DSM ACC3329 |
| | | | MSBI1 572-13-19 (Ab 10-3) | |
| | | | MSBI1 617-1-3 (Ab 11-5) | |
| **Group D** | speckles in cytoplasm | MSBI1 specific | D1: MSBI1 961-2-2 | DSM ACC3331 |
| | | | D2: MSBI1 761-5-1 (Ab 13) | DSM ACC3330 |

Anti-Rep antibodies of group A have an epitope within the amino acid sequence depicted in SEQ ID NO:3 (aa 198-217 of SEQ ID NO:1) and are capable of detecting MSBI1 Rep and Rep proteins comprising this conserved epitope of the Small Sphinx Genome group (e.g. MSBI2, CMI1, CMI4). In immunofluoresence assays such anti-Rep antibodies detect a specific Rep localisation pattern, wherein the main localisation is homogeneously distributed over the cytoplasm and nuclear membrane; and additional weak and homogeneously distributed localisation is seen in the nucleus. An example of such a group A antibody is antibody AB01 523-1-1 (also called antibody 1-5; DSM ACC3327) which was employed in the examples as group A antibody.

Anti-Rep antibodies of group B have an epitope within the amino acid sequence depicted in SEQ ID NO:2 (aa 33-50 of SEQ ID NO:1) and are capable of detecting MSBI1 Rep and Rep proteins comprising this conserved epitope of the Small Sphinx Genome group (e.g. MSBI2, CMI1, CMI4). In immunofluoresence assays such anti-Rep antibodies detect specifically speckles (cytoplasmatic aggregations) of the Rep protein (often in the periphery of the nuclear membrane). An example of such a group B antibody is the antibody designated as AB02 304-4-1 (also called antibody 5-2; DSM ACC3328) which was employed in the examples as group B antibody.

Anti-Rep antibodies of group C detect specifically a structural epitope of MSBI1 (SEQ ID NO:1). In immunofluoresence assays such anti-Rep antibodies detect a specific Rep localisation pattern, wherein the main localisation is homogeneously distributed over the cytoplasm and nuclear membrane; and additional weak and homogeneously distributed localisation is seen in the nucleus. An example of such a group C antibody is antibody MSBI1 381-6-2 (also called antibody 3-6; DSM ACC3329) which was employed in the Example as group C antibody with an epitope in the sequence of aa 137-324. Another example of an antibody of a group C antibody is antibody MBSI1 572-13-19 (also called antibody 10-3) detecting an epitope in the C-terminal domain of MSBI 1 Rep (aa 230-324). Another example of an antibody of a group C antibody is antibody MBSI1 617-1-3 (also called antibody 11-5) detecting an epitope in the N-terminal domain of MSBI 1 Rep (aa 1-136).

Anti-Rep antibodies of group D detect specifically a structural epitope of MSBI1 (SEQ ID NO:1), where antibody MSBI1 961-2-2 designated as "D1" (DSM ACC3331) detects an epitope depicted in SEQ ID NO:9 (aa 281-287) in the C-terminal domain of MSBI1. Antibody MSBI1 761-5-1 (also called antibody 13; DSM ACC3328) designated as "D2" detects a 3D structural epitope of MSBI1 which is exclusively accessible under in vivo conditions and is not accessible in Western Blots. In immunofluoresence assays such anti-Rep antibodies detect specifically speckles (cytoplasmatic aggregations) of the Rep protein (often in the periphery of the nuclear membrane.

The invention is further illustrated by, but not limited to, the following examples:

### Example 1: Detection of BMMF protein targets in prostate tissue

All tissue samples were provided by the tissue bank of the National Center for Tumor Diseases (NCT, Heidelberg, Germany and Institute of Pathology, Heidelberg University Hospital, Germany) in accordance with the regulations of the tissue bank and the approval of the ethics committee of Heidelberg University.

### Tissue staining

The paraffin-embedded tissue sections (∼4 µm thickness) were stained manually with EDTA epitope retrieval buffer and the given antibody incubations (c.f. Table). Detection was performed using Bond Polymer Refine Detection Kit (DS9800 Leica DAB Kit) including DAB chromogen and hematoxylin counterstain. Slides were scanned with a digital slide scanner (Hamamatsu) and analyzed based on Hamamatsu NDP viewer software.

| **Antibody** | **Source** | **Host** | **Dilution** | **Final concentration in µg/ml** | **Incubation time** |
|---|---|---|---|---|---|
| **Primary** | | | | | |
| Rep mAb #3-6 | T. Bund, DKFZ | mouse | 1:500 | 3.9 | 30 min at room temperature |
| Rep mAb #10-3 | T. Bund, DKFZ | mouse | 1:500 | 3.9 | |
| CD68 | Cell signaling #76437 | rabbit | 1:1000 | | |

| **Secondary** | | | | | |
|---|---|---|---|---|---|
| rabbit antimouse | Abcam #125904 | rabbit | 1:500 | | 20 min at room temperature |

Staining with anti-Rep antibodies (e.g. mAb 10-3, mAb 3-6) shows specific detection of protein targets in stromal tumor tissue regions within prostate cancer patient samples 17AD97 and 16RAV2 (Fig. 2 and 3). In general, the anti-Rep detection resulted in intense staining of smaller sized aggregates mainly within the cytoplasmic regions of cells within the stroma. Additionally, a colocalization of the anti-Rep stained signals with CD68-positive macrophages was observed. The regions with highest Rep-specific antibody detection correlate with regions with highest detection levels for CD68 positive cells pointing towards a localization of the Rep-specific antigens in inflammatory tissue areas, i.e. regions with especially high levels of inflammatory monocytes, circulating macrophages, or resident tissue macrophages. No signal detection was observed in control stainings with an antibody isotype control.

**SEQUENCE SUMMARY**

| SEQ ID NO | SEQUENCE |
|---|---|
| 1 | Amino acid sequence of Rep protein encoded by MSBI1.176 |
| | MSDLIVKDNALMNASYNLALVEQRLILLAIIEARETGKGINANDPLTVHASSYINQFN VERHTAYQALKDACKDLFARQFSYQEKRERGRINITSRWVSQIGYMDDTATVEIIFAP AVVPLITRLEEQFTQYDIEQISGLSSAYAVRMYELLICWRSTGKTPIIELDEFRKRIG VLDTEYTRTDNLKMRVIELALKQINEHTDITASYEQHKKGRVITGFSFKFKHKKQNSD KTPKNSDSSPRIVKHSQIPTNIVKQPENAKMSDLEHRASRVTGEIMRNRLSDRFKQGD ESAIDMMKRIQSEIITDAIADQWESKLEEFGVVF |
| 2 | Amino acid sequence of Rep peptide fragment |
| | EARETGKGINANDPLTVH |
| 3 | Amino acid sequence of Rep peptide fragment |
| | KQINEHTDITASYEOHKKGRT |
| 4 | His-Tag (with two neutral stuffer amino acids) |
| | GAHHHHHH |
| 5 | T7-Tag |
| | MASMTGGQQMG |
| 6 | FLAG-Tag |
| | DYKDDDDK |
| 7 | Strep-II-Tag |
| | WSHPQFEK |
| 8 | Amino acid sequence of Rep protein encoded by MSBI2.176 |
| | MSKLVVKDNALMNASYNLDLVEQRLILLAIIEARESGKGINANDPLTVHA ESYINQFGVHRVTAYQALKDACDNLFARQFSYQSKSEKGNIQNHRSRWVS EIIYIDTEATVKIIFAPAIVPLITRLEEQFTKYDIEQISDLSSAYAIRLY ELLICWRSTGKTPIIGLGEFRNRVGVLDSEYHRIAHLKERVIEHSIKQIN EHTDITATYEQHKKGRTITGFSFKFKQKKPKQAEIATETPKTATNDPDTT KPLTEPQIAKYSMILCKLGSISDLSNFPDYPAFANWIGNILRNPEKADEQ IAKRIFTALKTETDYSKKN |
| 9 | MSBI.1 specific epitope |
| | NRLSDRF |
| 10 | Amino acid sequence of Rep protein encoded by CMI1.252 |
| | MSDLIVKDNALMNASYNLALVEQRLILLAILEARETGKGINANDPLTVHASSYINQFN VERHTAYQALKDACKDLFARQFSYQEKRERGRINITSRWVSQIGYMDDTATVEIIFAP |
| | AVVPLITRLEEQFTQYDIEQISELSSAYAVRLYELLICWRSTGKTPIIDLTEFRKRLG VLDTEYTRTDNLKMRVIELGLKQINEHTDITASYEQHKKGRTITGFSFKFKQKKKTGA EMPKNSDSSPHIEKPSQIPANIAKQPENAKKDDLGHRASKITGLIMSNGLADRFKRGD ESVIDMMKRIKEEITTDTTADQWENKLEEFGVIFQS |
| 11 | Amino acid sequence of Rep protein encoded by CMI2.214 |
| | MSDLIVKDNALMNASYNLDLVEQRLILLAILEARETGKGINANDPLTVHAESYINQFG VARQTAYQALKDACKDLFARQFSYQEKRERGRANITSRWVSQIAYIDETATVEVIFAP AVVPLITRLEEQFTQYDIEQISGLSSAYAVRLYELLICWRSTGKTPVIELAEFRKRLG VLNDEYTRSDNFKKWIIENPIKQINEHTDITASYEQHKKGRTITGFSFKFKQKKKTEP ETPKNSDSSQRIEKPSQIPANIVKQPENANLSDLQHRASKITGLIMSNRLSDRFKQGD ESIMQMMARIQSEITTDSIADQWQSKLEEFGVVF |
| 12 | Amino acid sequence of Rep protein encoded by CMI3.168 |
| | MSDLIVKDNALMNASYNLALVEQRLILLAILEARETGKGINANDPLTVHASSYINQFN VERHTAYQALKDACKDLFARQFSYQEKRERGRANITSRWVSQIAYIDETATVEVIFAP AVVPLITRLEEQFTQYDIEQISGLSSAYAVRLYELLICWRTTGKTPVLDLTEFRKRLG VLDTEYTRTDNLKMRVIEQSLKQINKHTDITASYEQHKKGRTITGFSFKFKQKKKTEP ETPKNNDSGVSKPKTVEIPAEVVKQPKNTNLSDLEKRVRMITGAIAKNNLASRFQHGN ESPLDMMKRIQSEITSDETADLWQNKLESMGVVF |
| 13 | DNA sequence MSBI1 Rep codon-optimized |
| | ATGAGCGACCTGATCGTGAAAGACAATGCCCTGATGAACGCCTCCTACAACCTGGCAC TGGTCGAACAGAGACTGATTCTGCTGGCTATCATCGAGGCAAGGGAGACCGGCAAGGG CATCAACGCCAATGACCCCCTGACAGTGCACGCCAGCTCCTACATCAACCAGTTTAAT GTGGAGCGCCACACCGCCTATCAGGCCCTGAAGGACGCCTGCAAGGATCTGTTTGCCC GGCAGTTCAGCTACCAGGAGAAGCGGAGAGAGGCAGGATCAACATCACAAGCAGATG GGTGTCCCAGATCGGCTATATGGACGATACCGCCACAGTGGAGATCATCTTTGCACCA GCAGTGGTGCCTCTGATCACCAGGCTGGAGGAGCAGTTCACACAGTACGACATCGAGC AGATCTCCGGACTGTCTAGCGCCTACGCCGTGCGCATGTATGAGCTGCTGATCTGTTG GCGGTCTACCGGCAAGACACCTATCATCGAGCTGGATGAGTTCCGCAAGCGGATCGGC GTGCTGGACACCGAGTACACCAGAACAGATAACCTGAAGATGAGAGTGATCGAGCTGG CCCTGAAGCAGATCAATGAGCACACCGATATCACAGCCTCTTATGAGCAGCACAAGAA GGGCCGCGTGATCACCGGCTTCAGCTTTAAGTTCAAGCACAAGAAGCAGAACTCTGAC AAGACACCAAAGAATAGCGATTCCTCTCCCCGGATCGTGAAGCACAGCCAGATCCCTA CCAACATCGTGAAGCAGCCAGAGAATGCCAAGATGTCCGACCTGGAGCACAGGGCATC TAGGGTGACAGGCGAGATCATGAGAAATAGGCTGAGCGATCGGTTCAAGCAGGGCGAC GAGTCCGCCATCGATATGATGAAGAGAATCCAGTCCGAGATCATCACCGACGCCATCG CCGATCAGTGGGAATCTAAACTGGAAGAGTTTGGAGTCGTGTTTGGAGCACATCACCA TCATCATCACTGA |
| 14 | Protein sequence MSBI1 Rep codon-optimized |
| | MSDLIVKDNALMNASYNLALVEQRLILLAIIEARETGKGINANDPLTVHASSYINQFN |
| | VERHTAYQALKDACKDLFARQFSYQEKRERGRINITSRWVSQIGYMDDTATVEIIFAP AVVPLITRLEEQFTQYDIEQISGLSSAYAVRMYELLICWRSTGKTPIIELDEFRKRIG VLDTEYTRTDNLKMRVIELALKQINEHTDITASYEQHKKGRVITGFSFKFKHKKQNSD KTPKNSDSSPRIVKHSQIPTNIVKQPENAKMSDLEHRASRVTGEIMRNRLSDRFKQGD ESAIDMMKRIQSEIITDAIADQWESKLEEFGVVFGA |
| 15 | DNA sequence MSBI1 Rep wild-type |
| | ATGAGCGATTTAATAGTAAAAGATAACGCCCTAATGAATGCTAGTTATAACTTAGCTT TGGTTGAACAGAGGTTAATTCTATTAGCAATCATAGAAGCGAGAGAAACAGGCAAAGG GATTAATGCCAATGATCCTTTAACAGTTCATGCAAGTAGCTATATCAATCAATTTAAC GTAGAAAGGCATACGGCATATCAAGCCCTCAAAGATGCTTGTAAAGACTTGTTTGCCC GTCAATTCAGTTACCAAGAAAAGCGAGAACGAGGACGAATTAATATTACAAGTCGATG GGTTTCGCAAATTGGCTATATGGACGATACAGCAACCGTTGAGATTATTTTTGCCCCT GCGGTTGTTCCTCTGATTACACGGCTAGAGGAACAGTTCACCCAGTACGATATTGAGC AAATTAGCGGTTTATCGAGTGCATATGCTGTTCGTATGTACGAACTGCTGATTTGTTG GCGTAGCACAGGCAAAACACCAATTATTGAGCTAGACGAGTTTAGAAAGCGAATAGGT GTTTTAGATACTGAATACACTAGAACAGATAATTTAAAGATGCGAGTTATTGAATTAG CCCTAAAACAAATCAACGAACATACAGACATCACAGCAAGCTATGAACAACACAAAAA AGGGCGAGTGATTACAGGATTCTCATTCAAGTTTAAGCACAAGAAACAAAACAGCGAT AAAACGCCAAAAAATAGCGATTCTAGCCCACGTATCGTAAAACATAGTCAAATCCCTC CAACATTGTAAAACAGCCTGAAAACGCCAAAATGAGCGATTTAGAACATAGAGCGAGC CGTGTTACAGGGGAAATAATGCGAAATCGTCTGTCAGATCGGTTTAAACAAGGCGATG AATCAGCAATCGACATGATGAAACGTATTCAAAGTGAAATAATAACCGATGCAATAGC AGACCAGTGGGAAAGCAAACTGGAGGAGTTTGGCGTGGTTTTTTAG |

### REFERENCES:

Funk, M., et al. (2014). "Isolation of protein-associated circular DNA from healthy cattle serum". Genome Announc 2(4)
Giraldo, R., et al. (2011). "RepA-WH1 prionoid: a synthetic amyloid proteinopathy in a minimalist host." Prion 5(2):60-64
Gunst, K., et al. (2014). "Isolation of bacterial plasmid-related replication-associated cirular DNA from a serum sample of a multiple sclerosis patient." Genome Announc 2(4).
Lamberto, I., et al. (2014). "Mycovirus-like DNA virus sequences from cattle serum and human brain and serum samples from multiple sclerosis patients." Genome Announc 2(4).
Manuelidis L., 2011. "Nuclease resistant circular DNAs co-purify with infectivity in scrapie and CJD". J. Neurovirol. 17:131-145.
Torreira, E., et al. (2015). "Amyloidogenesis of bacterial prionoid RepA-WH1 recaptiulates dimer to monomer transitions of RepA in DNA replication initiation." Structure 23(1):183-189
Whitley, C., et al. (2014). "Novel replication-competent cirulara DNA molecules from healthy cattle serum and milk and multiple sclerosis-affected human brain tissue." Genome Announc 2(4).
zur Hausen, H., Bund, T., de Villiers, E.-M. (2017). "Infectious agents in bovine red meat and milk and their potential role in cancer and other chronic diseases." Curr. Top.Microbiol. Immunol., Volume 407, 83-116.

## Claims

1. Use of Bovine Meat and Milk Factor Group 1(BMMF1) Rep Protein as a biomarker for prostate cancer.

2. The use of claim 1 wherein the Rep protein is a MSBI1 genome-encoded Rep protein (MSBI1 Rep), a MSBI2 genome-encoded Rep protein (MSBI2 Rep), a CMI1 genome-encoded Rep protein (CMI1 Rep), a CMI2 genome-encoded Rep protein (CMI2 Rep) or CMI3 genome-encoded Rep protein (CMI3 Rep).

3. A method for providing a diagnosis or predisposition for prostate cancer in a subject, comprising the step of
detecting Rep protein in a sample from a subject by anti-Rep antibodies that bind to an epitope comprised by SEQ ID NO:2 or SEQ ID NO:3.

4. The method of claim 3, wherein the antibody specific for Rep protein binds to an epitope that is within an amino acid sequence selected from the group consisting of amino acids from 1 to 136, from 137 to 229 and from 230 to 324 of SEQ ID NO:1.

5. The method of claim 3 or 4, wherein the sample from a subject is selected from the group consisting of a cancerous prostate tissue, peripheral tissue surrounding the cancerous tissue, (benign) hyperplasias.

6. The method of any of claims 3 to 5, wherein additionally CD68 positive cells are detected in the sample by an anti-CD68 antibody.


SEQUENCE LISTING

<110>  Deutsches Krebsforschungszentrum

<120>  Use of BMMF Rep Protein as Biomarker for Prostate Cancer

<130>  K 3655EP

<150>
<151>

<160>  15

<170>  PatentIn version 3.5

<210>  1
<211>  324
<212>  PRT
<213>  artificial sequence

<220>
<223>  MSBI1 Rep protein

<400>  1

Met Ser Asp Leu Ile Val Lys Asp Asn Ala Leu Met Asn Ala Ser Tyr
1               5                   10                  15

Asn Leu Ala Leu Val Glu Gln Arg Leu Ile Leu Leu Ala Ile Ile Glu
            20                  25                  30

Ala Arg Glu Thr Gly Lys Gly Ile Asn Ala Asn Asp Pro Leu Thr Val
        35                  40                  45

His Ala Ser Ser Tyr Ile Asn Gln Phe Asn Val Glu Arg His Thr Ala
    50                  55                  60

Tyr Gln Ala Leu Lys Asp Ala Cys Lys Asp Leu Phe Ala Arg Gln Phe
65                  70                  75                  80

Ser Tyr Gln Glu Lys Arg Glu Arg Gly Arg Ile Asn Ile Thr Ser Arg
                85                  90                  95

Trp Val Ser Gln Ile Gly Tyr Met Asp Asp Thr Ala Thr Val Glu Ile
            100                 105                 110

Ile Phe Ala Pro Ala Val Val Pro Leu Ile Thr Arg Leu Glu Glu Gln
        115                 120                 125

Phe Thr Gln Tyr Asp Ile Glu Gln Ile Ser Gly Leu Ser Ser Ala Tyr
    130                 135                 140

Ala Val Arg Met Tyr Glu Leu Leu Ile Cys Trp Arg Ser Thr Gly Lys
145                 150                 155                 160

Thr Pro Ile Ile Glu Leu Asp Glu Phe Arg Lys Arg Ile Gly Val Leu
                165                 170                 175

Asp Thr Glu Tyr Thr Arg Thr Asp Asn Leu Lys Met Arg Val Ile Glu
            180                 185                 190

Leu Ala Leu Lys Gln Ile Asn Glu His Thr Asp Ile Thr Ala Ser Tyr
        195                 200                 205

Glu Gln His Lys Lys Gly Arg Val Ile Thr Gly Phe Ser Phe Lys Phe
    210                 215                 220

Lys His Lys Lys Gln Asn Ser Asp Lys Thr Pro Lys Asn Ser Asp Ser
225                 230                 235                 240

Ser Pro Arg Ile Val Lys His Ser Gln Ile Pro Thr Asn Ile Val Lys
                245                 250                 255

Gln Pro Glu Asn Ala Lys Met Ser Asp Leu Glu His Arg Ala Ser Arg
            260                 265                 270

Val Thr Gly Glu Ile Met Arg Asn Arg Leu Ser Asp Arg Phe Lys Gln
        275                 280                 285

Gly Asp Glu Ser Ala Ile Asp Met Met Lys Arg Ile Gln Ser Glu Ile
    290                 295                 300

Ile Thr Asp Ala Ile Ala Asp Gln Trp Glu Ser Lys Leu Glu Glu Phe
305                 310                 315                 320

Gly Val Val Phe

<210>  2
<211>  18
<212>  PRT
<213>  artificial sequence

<220>
<223>  Rep peptide

<400>  2

Glu Ala Arg Glu Thr Gly Lys Gly Ile Asn Ala Asn Asp Pro Leu Thr
1               5                   10                  15

Val His

<210>  3
<211>  20
<212>  PRT
<213>  artificial sequence

<220>
<223>  Rep peptide

<400>  3

Lys Gln Ile Asn Glu His Thr Asp Ile Thr Ala Ser Tyr Glu His Lys
1               5                   10                  15


Lys Gly Arg Thr
            20


<210>  4
<211>  8
<212>  PRT
<213>  artificial sequence

<220>
<223>  His tag

<400>  4

Gly Ala His His His His His His
1               5


<210>  5
<211>  11
<212>  PRT
<213>  artificial sequence

<220>
<223>  T7 tag

<400>  5

Met Ala Ser Met Thr Gly Gly Gln Gln Met Gly
1               5                   10


<210>  6
<211>  8
<212>  PRT
<213>  artificial sequence

<220>
<223>  FLAG tag

<400>  6

Asp Tyr Lys Asp Asp Asp Asp Lys
1               5


<210>  7
<211>  8

<212>  PRT
<213>  artificial sequence

<220>
<223>  Trep II tag

<400>  7

Trp Ser His Pro Gln Phe Glu Lys
1               5


<210>  8
<211>  319
<212>  PRT
<213>  artificial sequence

<220>
<223>  MSBI2.176

<400>  8

Met Ser Lys Leu Val Val Lys Asp Asn Ala Leu Met Asn Ala Ser Tyr
1               5                   10                  15

Asn Leu Asp Leu Val Glu Gln Arg Leu Ile Leu Leu Ala Ile Ile Glu
            20                  25                  30

Ala Arg Glu Ser Gly Lys Gly Ile Asn Ala Asn Asp Pro Leu Thr Val
        35                  40                  45

His Ala Glu Ser Tyr Ile Asn Gln Phe Gly Val His Arg Val Thr Ala
    50                  55                  60

Tyr Gln Ala Leu Lys Asp Ala Cys Asp Asn Leu Phe Ala Arg Gln Phe
65                  70                  75                  80

Ser Tyr Gln Ser Lys Ser Glu Lys Gly Asn Ile Gln Asn His Arg Ser
                85                  90                  95

Arg Trp Val Ser Glu Ile Ile Tyr Ile Asp Thr Glu Ala Thr Val Lys
            100                 105                 110

Ile Ile Phe Ala Pro Ala Ile Val Pro Leu Ile Thr Arg Leu Glu Glu
        115                 120                 125

Gln Phe Thr Lys Tyr Asp Ile Glu Gln Ile Ser Asp Leu Ser Ser Ala
    130                 135                 140

Tyr Ala Ile Arg Leu Tyr Glu Leu Leu Ile Cys Trp Arg Ser Thr Gly
145                 150                 155                 160

Lys Thr Pro Ile Ile Gly Leu Gly Glu Phe Arg Asn Arg Val Gly Val

165                 170                 175

Leu Asp Ser Glu Tyr His Arg Ile Ala His Leu Lys Glu Arg Val Ile
            180                 185                 190

Glu His Ser Ile Lys Gln Ile Asn Glu His Thr Asp Ile Thr Ala Thr
        195                 200                 205

Tyr Glu Gln His Lys Lys Gly Arg Thr Ile Thr Gly Phe Ser Phe Lys
    210                 215                 220

Phe Lys Gln Lys Lys Pro Lys Gln Ala Glu Ile Ala Thr Glu Thr Pro
225                 230                 235                 240

Lys Thr Ala Thr Asn Asp Pro Asp Thr Thr Lys Pro Leu Thr Glu Pro
                245                 250                 255

Gln Ile Ala Lys Tyr Ser Met Ile Leu Cys Lys Leu Gly Ser Ile Ser
            260                 265                 270

Asp Leu Ser Asn Phe Pro Asp Tyr Pro Ala Phe Ala Asn Trp Ile Gly
        275                 280                 285

Asn Ile Leu Arg Asn Pro Glu Lys Ala Asp Glu Gln Ile Ala Lys Arg
    290                 295                 300

Ile Phe Thr Ala Leu Lys Thr Glu Thr Asp Tyr Ser Lys Lys Asn
305                 310                 315

<210>  9
<211>  7
<212>  PRT
<213>  artificial sequence

<220>
<223>  MSBI1 epitope

<400>  9

Asn Arg Leu Ser Asp Arg Phe
1               5

<210>  10
<211>  326
<212>  PRT
<213>  artificial

<220>
<223>  CMI1.252

<400>  10

Met Ser Asp Leu Ile Val Lys Asp Asn Ala Leu Met Asn Ala Ser Tyr
1               5                   10                  15

Asn Leu Ala Leu Val Glu Gln Arg Leu Ile Leu Leu Ala Ile Leu Glu
            20                  25                  30

Ala Arg Glu Thr Gly Lys Gly Ile Asn Ala Asn Asp Pro Leu Thr Val
        35                  40                  45

His Ala Ser Ser Tyr Ile Asn Gln Phe Asn Val Glu Arg His Thr Ala
    50                  55                  60

Tyr Gln Ala Leu Lys Asp Ala Cys Lys Asp Leu Phe Ala Arg Gln Phe
65                  70                  75                  80

Ser Tyr Gln Glu Lys Arg Glu Arg Gly Arg Ile Asn Ile Thr Ser Arg
                85                  90                  95

Trp Val Ser Gln Ile Gly Tyr Met Asp Asp Thr Ala Thr Val Glu Ile
            100                 105                 110

Ile Phe Ala Pro Ala Val Val Pro Leu Ile Thr Arg Leu Glu Glu Gln
        115                 120                 125

Phe Thr Gln Tyr Asp Ile Glu Gln Ile Ser Glu Leu Ser Ser Ala Tyr
    130                 135                 140

Ala Val Arg Leu Tyr Glu Leu Leu Ile Cys Trp Arg Ser Thr Gly Lys
145                 150                 155                 160

Thr Pro Ile Ile Asp Leu Thr Glu Phe Arg Lys Arg Leu Gly Val Leu
                165                 170                 175

Asp Thr Glu Tyr Thr Arg Thr Asp Asn Leu Lys Met Arg Val Ile Glu
            180                 185                 190

Leu Gly Leu Lys Gln Ile Asn Glu His Thr Asp Ile Thr Ala Ser Tyr
        195                 200                 205

Glu Gln His Lys Lys Gly Arg Thr Ile Thr Gly Phe Ser Phe Lys Phe
    210                 215                 220

Lys Gln Lys Lys Lys Thr Gly Ala Glu Met Pro Lys Asn Ser Asp Ser
225                 230                 235                 240

Ser Pro His Ile Glu Lys Pro Ser Gln Ile Pro Ala Asn Ile Ala Lys
                245                 250                 255

Gln Pro Glu Asn Ala Lys Lys Asp Asp Leu Gly His Arg Ala Ser Lys
                260                 265                 270

Ile Thr Gly Leu Ile Met Ser Asn Gly Leu Ala Asp Arg Phe Lys Arg
        275                 280                 285

Gly Asp Glu Ser Val Ile Asp Met Met Lys Arg Ile Lys Glu Glu Ile
    290                 295                 300

Thr Thr Asp Thr Thr Ala Asp Gln Trp Glu Asn Lys Leu Glu Glu Phe
305                 310                 315                 320

Gly Val Ile Phe Gln Ser
                325

<210>  11
<211>  324
<212>  PRT
<213>  artificial

<220>
<223>  CMI2.214

<400>  11

Met Ser Asp Leu Ile Val Lys Asp Asn Ala Leu Met Asn Ala Ser Tyr
1               5                   10                  15

Asn Leu Asp Leu Val Glu Gln Arg Leu Ile Leu Leu Ala Ile Leu Glu
            20                  25                  30

Ala Arg Glu Thr Gly Lys Gly Ile Asn Ala Asn Asp Pro Leu Thr Val
        35                  40                  45

His Ala Glu Ser Tyr Ile Asn Gln Phe Gly Val Ala Arg Gln Thr Ala
    50                  55                  60

Tyr Gln Ala Leu Lys Asp Ala Cys Lys Asp Leu Phe Ala Arg Gln Phe
65                  70                  75                  80

Ser Tyr Gln Glu Lys Arg Glu Arg Gly Arg Ala Asn Ile Thr Ser Arg
                85                  90                  95

Trp Val Ser Gln Ile Ala Tyr Ile Asp Glu Thr Ala Thr Val Glu Val
            100                 105                 110

Ile Phe Ala Pro Ala Val Val Pro Leu Ile Thr Arg Leu Glu Glu Gln
        115                 120                 125

Phe Thr Gln Tyr Asp Ile Glu Gln Ile Ser Gly Leu Ser Ser Ala Tyr
    130                 135                 140

Ala Val Arg Leu Tyr Glu Leu Leu Ile Cys Trp Arg Ser Thr Gly Lys
145                 150                 155                 160

Thr Pro Val Ile Glu Leu Ala Glu Phe Arg Lys Arg Leu Gly Val Leu
                165                 170                 175

Asn Asp Glu Tyr Thr Arg Ser Asp Asn Phe Lys Lys Trp Ile Ile Glu
            180                 185                 190

Asn Pro Ile Lys Gln Ile Asn Glu His Thr Asp Ile Thr Ala Ser Tyr
        195                 200                 205

Glu Gln His Lys Lys Gly Arg Thr Ile Thr Gly Phe Ser Phe Lys Phe
    210                 215                 220

Lys Gln Lys Lys Lys Thr Glu Pro Glu Thr Pro Lys Asn Ser Asp Ser
225                 230                 235                 240

Ser Gln Arg Ile Glu Lys Pro Ser Gln Ile Pro Ala Asn Ile Val Lys
                245                 250                 255

Gln Pro Glu Asn Ala Asn Leu Ser Asp Leu Gln His Arg Ala Ser Lys
            260                 265                 270

Ile Thr Gly Leu Ile Met Ser Asn Arg Leu Ser Asp Arg Phe Lys Gln
        275                 280                 285

Gly Asp Glu Ser Ile Met Gln Met Met Ala Arg Ile Gln Ser Glu Ile
    290                 295                 300

Thr Thr Asp Ser Ile Ala Asp Gln Trp Gln Ser Lys Leu Glu Glu Phe
305                 310                 315                 320

Gly Val Val Phe

<210>  12
<211>  324
<212>  PRT
<213>  artificial

<220>
<223>  CMI3.168

<400>  12

Met Ser Asp Leu Ile Val Lys Asp Asn Ala Leu Met Asn Ala Ser Tyr

1               5                   10                  15

Asn Leu Ala Leu Val Glu Gln Arg Leu Ile Leu Leu Ala Ile Leu Glu
            20                  25                  30

Ala Arg Glu Thr Gly Lys Gly Ile Asn Ala Asn Asp Pro Leu Thr Val
        35                  40                  45

His Ala Ser Ser Tyr Ile Asn Gln Phe Asn Val Glu Arg His Thr Ala
    50                  55                  60

Tyr Gln Ala Leu Lys Asp Ala Cys Lys Asp Leu Phe Ala Arg Gln Phe
65                  70                  75                  80

Ser Tyr Gln Glu Lys Arg Glu Arg Gly Arg Ala Asn Ile Thr Ser Arg
                85                  90                  95

Trp Val Ser Gln Ile Ala Tyr Ile Asp Glu Thr Ala Thr Val Glu Val
            100                 105                 110

Ile Phe Ala Pro Ala Val Val Pro Leu Ile Thr Arg Leu Glu Glu Gln
        115                 120                 125

Phe Thr Gln Tyr Asp Ile Glu Gln Ile Ser Gly Leu Ser Ser Ala Tyr
    130                 135                 140

Ala Val Arg Leu Tyr Glu Leu Leu Ile Cys Trp Arg Thr Thr Gly Lys
145                 150                 155                 160

Thr Pro Val Leu Asp Leu Thr Glu Phe Arg Lys Arg Leu Gly Val Leu
                165                 170                 175

Asp Thr Glu Tyr Thr Arg Thr Asp Asn Leu Lys Met Arg Val Ile Glu
            180                 185                 190

Gln Ser Leu Lys Gln Ile Asn Lys His Thr Asp Ile Thr Ala Ser Tyr
        195                 200                 205

Glu Gln His Lys Lys Gly Arg Thr Ile Thr Gly Phe Ser Phe Lys Phe
    210                 215                 220

Lys Gln Lys Lys Lys Thr Glu Pro Glu Thr Pro Lys Asn Asn Asp Ser
225                 230                 235                 240

Gly Val Ser Lys Pro Lys Thr Val Glu Ile Pro Ala Glu Val Val Lys
                245                 250                 255

Gln Pro Lys Asn Thr Asn Leu Ser Asp Leu Glu Lys Arg Val Arg Met
            260                 265                 270

Ile Thr Gly Ala Ile Ala Lys Asn Asn Leu Ala Ser Arg Phe Gln His
        275                 280                 285

Gly Asn Glu Ser Pro Leu Asp Met Met Lys Arg Ile Gln Ser Glu Ile
    290                 295                 300

Thr Ser Asp Glu Thr Ala Asp Leu Trp Gln Asn Lys Leu Glu Ser Met
305                 310                 315                 320

Gly Val Val Phe

<210>  13
<211>  999
<212>  DNA
<213>  artificial sequence

<220>
<223>  codon-optimized MSBI1

<400>  13
atgagcgacc tgatcgtgaa agacaatgcc ctgatgaacg cctcctacaa cctggcactg      60

gtcgaacaga gactgattct gctggctatc atcgaggcaa gggagaccgg caagggcatc     120

aacgccaatg accccctgac agtgcacgcc agctcctaca tcaaccagtt taatgtggag     180

cgccacaccg cctatcaggc cctgaaggac gcctgcaagg atctgtttgc ccggcagttc     240

agctaccagg agaagcggga gagaggcagg atcaacatca caagcagatg ggtgtcccag     300

atcggctata tggacgatac cgccacagtg gagatcatct ttgcaccagc agtggtgcct     360

ctgatcacca ggctggagga gcagttcaca cagtacgaca tcgagcagat ctccggactg     420

tctagcgcct acgccgtgcg catgtatgag ctgctgatct gttggcggtc taccggcaag     480

acacctatca tcgagctgga tgagttccgc aagcggatcg gcgtgctgga caccgagtac     540

accagaacag ataacctgaa gatgagagtg atcgagctgg ccctgaagca gatcaatgag     600

cacaccgata tcacagcctc ttatgagcag cacaagaagg gccgcgtgat caccggcttc     660

agctttaagt tcaagcacaa gaagcagaac tctgacaaga caccaaagaa tagcgattcc     720

tctccccgga tcgtgaagca cagccagatc cctaccaaca tcgtgaagca gccagagaat     780

gccaagatgt ccgacctgga gcacagggca tctagggtga caggcgagat catgagaaat     840

aggctgagcg atcggttcaa gcagggcgac gagtccgcca tcgatatgat gaagagaatc     900

cagtccgaga tcatcaccga cgccatcgcc gatcagtggg aatctaaact ggaagagttt     960

ggagtcgtgt ttggagcaca tcaccatcat catcactga                            999

<210>  14
<211>  326
<212>  PRT
<213>  artificial sequence

<220>
<223>  MSBI1 protein

<400>  14

Met Ser Asp Leu Ile Val Lys Asp Asn Ala Leu Met Asn Ala Ser Tyr
1               5                   10                  15

Asn Leu Ala Leu Val Glu Gln Arg Leu Ile Leu Leu Ala Ile Ile Glu
            20                  25                  30

Ala Arg Glu Thr Gly Lys Gly Ile Asn Ala Asn Asp Pro Leu Thr Val
        35                  40                  45

His Ala Ser Ser Tyr Ile Asn Gln Phe Asn Val Glu Arg His Thr Ala
    50                  55                  60

Tyr Gln Ala Leu Lys Asp Ala Cys Lys Asp Leu Phe Ala Arg Gln Phe
65                  70                  75                  80

Ser Tyr Gln Glu Lys Arg Glu Arg Gly Arg Ile Asn Ile Thr Ser Arg
                85                  90                  95

Trp Val Ser Gln Ile Gly Tyr Met Asp Asp Thr Ala Thr Val Glu Ile
            100                 105                 110

Ile Phe Ala Pro Ala Val Val Pro Leu Ile Thr Arg Leu Glu Glu Gln
        115                 120                 125

Phe Thr Gln Tyr Asp Ile Glu Gln Ile Ser Gly Leu Ser Ser Ala Tyr
    130                 135                 140

Ala Val Arg Met Tyr Glu Leu Leu Ile Cys Trp Arg Ser Thr Gly Lys
145                 150                 155                 160

Thr Pro Ile Ile Glu Leu Asp Glu Phe Arg Lys Arg Ile Gly Val Leu
                165                 170                 175

Asp Thr Glu Tyr Thr Arg Thr Asp Asn Leu Lys Met Arg Val Ile Glu
            180                 185                 190

Leu Ala Leu Lys Gln Ile Asn Glu His Thr Asp Ile Thr Ala Ser Tyr
        195                 200                 205

Glu Gln His Lys Lys Gly Arg Val Ile Thr Gly Phe Ser Phe Lys Phe
    210                 215                 220

Lys His Lys Lys Gln Asn Ser Asp Lys Thr Pro Lys Asn Ser Asp Ser
225                 230                 235                 240

Ser Pro Arg Ile Val Lys His Ser Gln Ile Pro Thr Asn Ile Val Lys
                245                 250                 255

Gln Pro Glu Asn Ala Lys Met Ser Asp Leu Glu His Arg Ala Ser Arg
            260                 265                 270

Val Thr Gly Glu Ile Met Arg Asn Arg Leu Ser Asp Arg Phe Lys Gln
        275                 280                 285

Gly Asp Glu Ser Ala Ile Asp Met Met Lys Arg Ile Gln Ser Glu Ile
    290                 295                 300

Ile Thr Asp Ala Ile Ala Asp Gln Trp Glu Ser Lys Leu Glu Glu Phe
305                 310                 315                 320

Gly Val Val Phe Gly Ala
                325

<210>  15
<211>  974
<212>  DNA
<213>  artificial sequence

<220>
<223>  MSBI1 wild type

<400>  15
atgagcgatt taatagtaaa agataacgcc ctaatgaatg ctagttataa cttagctttg      60

gttgaacaga ggttaattct attagcaatc atagaagcga gagaaacagg caaagggatt     120

aatgccaatg atcctttaac agttcatgca agtagctata tcaatcaatt taacgtagaa     180

aggcatacgg catatcaagc cctcaaagat gcttgtaaag acttgtttgc ccgtcaattc     240

agttaccaag aaaagcgaga acgaggacga attaatatta caagtcgatg ggtttcgcaa     300

attggctata tggacgatac agcaaccgtt gagattattt ttgcccctgc ggttgttcct     360

ctgattacac ggctagagga acagttcacc cagtacgata ttgagcaaat tagcggttta     420

tcgagtgcat atgctgttcg tatgtacgaa ctgctgattt gttggcgtag cacaggcaaa     480

acaccaatta ttgagctaga cgagtttaga aagcgaatag gtgttttaga tactgaatac     540

actagaacag ataatttaaa gatgcgagtt attgaattag ccctaaaaca aatcaacgaa     600

catacagaca tcacagcaag ctatgaacaa cacaaaaaag ggcgagtgat tacaggattc     660

tcattcaagt ttaagcacaa gaaacaaaac agcgataaaa cgccaaaaaa tagcgattct 720

agcccacgta tcgtaaaaca tagtcaaatc cctccaacat tgtaaaacag cctgaaaacg 780

ccaaaatgag cgatttagaa catagagcga gccgtgttac aggggaaata atgcgaaatc 840

gtctgtcaga tcggtttaaa caaggcgatg aatcagcaat cgacatgatg aaacgtattc 900

aaagtgaaat aataaccgat gcaatagcag accagtggga aagcaaactg gaggagtttg 960

gcgtggtttt ttag 974